# EUROPEAN PATENT SPECIFICATION

(11) **EP 0 431 905 B1**
(45) Date of publication and mention of the grant of the patent: **11.05.1994**
(21) Application number: 90313173.8
(22) Date of filing: 05.12.1990
(51) Int. Cl.: C12N 15/10, C12P 19/34

(54) **Method for purifying phage DNA**
Verfahren zur Reinigung von Phagen-DNA
Méthode pour la purification de DNA phagique

(30) Priority: 06.12.1989 JP 317114/89
(43) Date of publication of application: 12.06.1991
(73) Proprietor: Tosoh Corporation, Shinnanyo-shi, Yamaguchi-ken, 746 (JP); RIKEN INSTITUTE OF PHYSICAL AND CHEMICAL RESEARCH, Wako-shi, Saitama-ken, 351-01 (JP)
(72) Inventor: Ogawa, Kazuo, Murayama-shi, Tokyo 189 (JP); Nishi, Akihiro, Ebina-shi, Kanagawa-ken 243-04 (JP); Koyama, Kenji, Atsugi-shi, Kanagawa-ken 243 (JP); Matsuno, Takanori, Ebina-shi, Kanagawa-ken 243-04 (JP)
(74) Representative: Kearney, Kevin David Nicholas

(56) References cited:
- EP-A- 0 240 191
- EP-A- 0 376 080
- WO-A-89/01035

## Description

### BACKGROUND OF THE INVENTION

### I. Field of the Invention

The present invention relates to a method for purifying phage DNA. The method of the present invention may be used in the field of genetic engineering.

### II. Description of the Related Art

Recently, with the progress of the genetic engineering techniques, nucleic acids are widely purified from various cells, viruses and phages. For determining base sequences of DNAs carrying genetic information, it is an important technique to replicate single-stranded DNAs by culturing M13 phage with *E*. *coli*. Purification of phage DNAs is generally carried out in this technique. The widely adopted conventional method for purifying phage DNAs from the culture medium comprises the steps of removing *E*. *coli* cells from the culture medium by centrifugation, precipitating the phage by treatment with polyethyleneglycol, removing proteins by phenol extraction and concentrating DNA by ethanol precipitation.

However, with this conventional method, reagents which are toxic to human, such as phenol and chloroform must be used. Further, since centrifugation is necessary in each step, it is difficult to automatize the method. For automatizing the purification, a method which does not require centrifugation is desired.

### SUMMARY OF THE INVENTION

Accordingly, the object of the present invention is to provide a process of purifying phage DNAs by which phage DNAs may be purified to high purity without centrifugation steps and without using reagents toxic to human.

The present inventors intensively studied to find that phage DNAs may be purified to high purity by removing *E*. *coli* cells by filtration through a membrane filter, decomposing and denaturing the phage proteins, and then removing the proteins by ultrafiltration, to complete the present invention.

That is, the present invention provides a method for purifying phage DNAs comprising, in the order mentioned, the steps of filtering a mixture containing microorganism cells and phage through a membrane filter to obtain a filtrate, so as to remove said microorganism cells; decomposing and denaturing phage proteins in said filtrate; and subjecting the resultant to ultrafiltration so as to remove impurities.

By the method of the present invention, phage DNAs may be purified to high purity with high yield from a culture medium without conducting a centrifugation step. According to the method of the present invention, since the centrifugation steps is not necessary, the method may be automatized. Further, with the method of the present invention, toxic reagents are not necessary and the time required for the purification is shortened.

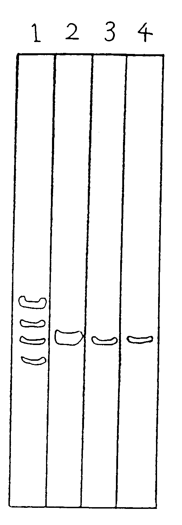

### BRIEF DESCRIPTION OF THE DRAWING

The drawing shows an agarose gel electrophoresis pattern of a phage DNA purified by the method of the present invention together with an electrophoresis pattern of the phage DNA purified by the conventional method and an electrophoresis pattern of a commercially available phage DNA.

### BEST MODE FOR CARRYING OUT THE INVENTION

In the method of the present invention, a mixture containing microorganism cells and phage is treated. Representative example of such a mixture is a culture medium of a microorganism which is infected with a virus. The culture medium may be treated as it is by the method of the present invention. For example, a culture medium containing M13 phage and *E*. *coli* in 2 x TY medium or the like may be treated by the method of the present invention.

In the first step of the method of the present invention, the above-described mixture is filtered through a membrane filter so as to remove microorganism cells. The pore size of the membrane filter used here may preferably be 0.45 - 0.22 µm. The material constituting the membrane filter is not at all restricted.

In the second step, the phage proteins in the filtrate is decomposed and denatured. The decomposition and denaturation of the proteins may be carried out by treating the filtrate with a proteolytic enzyme such as Proteinase K. In this case, the concentration of the proteolytic enzyme may be appropriately selected. When a culture medium containing M13 phage and *E*. *coli* is treated, the concentration of the proteolytic enzyme may usually be 0.001 - 0.5 mg/ml. As described later, it is preferred to subject the filtrate to ultrafiltration before the treatment with a proteolytic enzyme and to add the proteolytic enzyme to the phage remained on the ultrafiltration membrane, although this ultrafiltration step is not required.

Other than the treatment with a proteolytic enzyme, the decomposition and denaturation of the phage proteins may be carried out by treatment with an organic solvent, a surfactant or an alkali or by heat treatment or the like. More particularly, the step of decomposing and denaturing the proteins may be carried out by treating the protein with an organic solvent such as 30 - 100% methanol or ethanol. The separation of DNA and proteins may be attained by using a reagent which does not have a decomposing ability as strong as proteolytic enzymes or organic solvents, when a comparatively simple phage is to be treated. For example, the decomposition and denaturation of proteins may be attained by treatment with a surfactant. Preferred examples of the surfactant include anion surfactants such as sodium lauryl sulfate (SDS). In case of using SDS, the concentration thereof may preferably be 0.01 - 1% by weight. Since proteins are weak to heat and nucleic acids such as DNAs are relatively strong to heat, heat may be utilized for the protein-nucleic acid separation. In this case, the heat treatment may preferably be carried out at a temperature of 80 - 100°C for 5 - 20 minutes. The decomposition and denaturation of the proteins may also be carried out by alkali treatment. Preferred examples of the alkali include aqueous solution of alkaline metal hydroxides with a concentration of 0.1 - 1N.

The above-mentioned treatments may be carried out individually or in combination. By this step, proteins can be removed from the phage DNA.

The resulting solution is then ultrafiltered. The ultrafiltration membrane used herein preferably has a fractionation molecular weight of 20,000 to 1,000,000. If the molecular weight to be fractionated is smaller than this range, the efficiency of the removal of proteins is reduced. On the other hand, if the molecular weight is larger than this range, DNA may pass through the ultrafilter. The ultrafilter may be made of any material. For example, commercially available ultrafilters made of polysulfone may be employed. After the ultrafiltration, by washing the ultrafilter with an appropriate solution such as a buffer solution, the phage DNA may be recovered as a solution. To promote the yield of the phage DNA, it is preferred to wash the ultrafilter by passing the solution through the ultrafilter. The solution to be used for washing the ultrafilter is not restricted. Widely used buffer solutions such as TE-buffer (Tris-HCl buffer containing EDTA) may be employed as the solution for washing the ultrafilter. By this ultrafiltration step, the proteins and other impurities are removed from the solution obtained in the second step, so that phage DNA is purified.

The second step may be carried out immediately after the first step as described above. However, after the first step, the obtained filtrate may be subjected to ultrafiltration to remove low molecular components, thereby purifying the phage. In this case, the ultrafiltration may be carried out in the same manner as in the third step just described above. Further, if this ultrafiltration step is employed, the treatment for decomposing and denaturing the proteins in the second step may be carried out on the ultrafilter, and the ultrafiltration in the third step may be carried out on the same ultrafilter.

### Example

The invention will now be described by way of an example thereof. The example is presented for illustration purpose only and should not be interpreted in any restrictive way.

*E*. *coli* JM109 was inoculated to 2 x TY medium, and then M13 phage was infected thereto. The medium was incubated at 37°C for 4 hours.

The resulting culture medium was filtered through a membrane filter having a pore size of 0.45 µm under a pressure with nitrogen gas.

The obtained filtrate was subjected to ultrafiltration through a ultrafilter having a fractionation molecular weight of 300,000 to remove low molecular weight components in the culture medium. To decompose the proteins of the phage, 5 µg/µl solution of proteinase K was placed on the ultrafilter, and the reaction was allowed to occur for 10 minutes.

The enzyme solution was removed under pressure, and the ultrafilter was washed with TE buffer by passing through the buffer under pressure through the ultrafilter. Two hundred microliters of TE buffer was placed on the ultrafilter and the ultrafilter was shaken. Thereafter, the TE buffer was recovered by using a pipette.

By this procedure, 2.5 µg of phage DNA was recovered from 2 ml of the culture medium.

The thus obtained phage DNA was subjected to agarose gel electrophoresis according to a conventional method. As controls, commercially available M13 phage DNA and the M13 phage DNA purified by the conventional purification method comprising polyethylene glycol precipitation and phenol extraction were also subjected to agarose gel electrophoresis.

The results are shown in the drawing. In the drawing, lane 1 shows an electrophoresis pattern of molecular weight markers consisting of *Hin*dIII digest of λ phage, lane 2 shows an electrophoresis pattern of commercially available M13 phage single-stranded DNA, lane 3 shows an electrophoresis pattern of M13 phage DNA purified by the conventional polyethylene glycol precipitation and phenol extraction, and lane 4 shows an electrophoresis pattern of M13 phage DNA purified by the method of the present invention.

As is apparent from the drawing, it was confirmed that the phage DNA was purified to high purity by the method of the present invention.

## Claims

1. A method for purifying phage DNA comprising, in the order mentioned, the steps of:
filtering a mixture containing microorganism cells and phage through a membrane filter to obtain a filtrate, so as to remove the said microorganism cells;
decomposing and denaturing phage proteins in the said filtrate; and
subjecting the resultant to ultrafiltration so as to remove impurities.

2. A method as claimed in Claim 1, characterised in that the pore size of the said membrane filter is 0.45 µm to 0.22 µm.

3. A method as claimed in Claim 1 or Claim 2 characterised in that the molecular weight to be fractionated by the said ultrafiltration is in the range 20,000 to 1,000,000.

4. A method as claimed in Claim 1, 2 or 3 characterised in that the step of decomposing and denaturing phage proteins is carried out by treating the said phage proteins with a proteolytic enzyme.

5. A method as claimed in Claim 1, 2, 3 or 4 characterised in that it further comprises the step of ultrafiltration of the said filtrate obtained in the first step from the membrane filter before the said step of decomposing and denaturing the phage proteins.

## Patentansprüche

1. Verfahren zur Reinigung von Phagen-DNA, das die folgenden Schritte in der angegebenen Reihenfolge umfaßt:
Filtern eines Gemisches, das Mikroorganismuszellen und Phagen enthält, durch ein Membranfilter zur Gewinnung eines Filtrats, wobei die Mikroorganismuszellen entfernt werden;
Zersetzung und Denaturierung der Phagenproteine in dem Filtrat; und
Durchführung einer Ultrafiltration mit dem erhaltenen Produkt, um Verunreinigungen zu entfernen.

2. Verfahren nach Anspruch 1, dadurch gekennzeichnet, daß die Porengröße des Membranfilters 0,45 µm bis 0,22 µm beträgt.

3. Verfahren nach Anspruch 1 oder 2, dadurch gekennzeichnet, daß das durch die Ultrafiltration zu fraktionierende Molekulargewicht im Bereich 20 000 bis 1 000 000 liegt.

4. Verfahren nach Anspruch 1, 2 oder 3, dadurch gekennzeichnet, daß der Zersetzungs- und Denaturierungsschritt der Phagenproteine durch Behandlung der Phagenproteine mit einem proteolytischen Enzym durchgeführt wird.

5. Verfahren nach Anspruch 1, 2, 3 oder 4, dadurch gekennzeichnet, daß es als weiteren Schritt die Ultrafiltration des im ersten Schritt vom Membranfilter erhaltenen Filtrats vor dem Zersetzungs- und Denaturierungsschritt der Phagenproteine umfaßt.

## Revendications

1. Procédé de purification d'ADN phagique comprenant,dans l'ordre mentionné,les étapes de:
filtration d'un mélange contenant des cellules de microorganismes et un phage à travers un filtre à membrane pour obtenir un filtrat, afin d'enlever lesdites cellules de microorganismes;
décomposition et dénaturation des protéines phagiques dans ledit filtrat; et
soumission du produit résultant à une ultrafiltration de manière à enlever les impuretés.

2. Procédé selon la revendication 1, caractérisé en ce que la taille des pores dudit filtre à membrane de 0,45 µm à 0,22 µm .

3. Procédé selon la revendication 1 ou la revendication 2, caractérisé en ce que le poids moléculaire à fractionner par ladite ultrafiltration est dans un intervalle allant de 20000 à 1000000 .

4. Procédé selon la revendication 1, 2 ou 3, caractérisé en ce que l'étape de décomposition et de dénaturation des protéines phagiques est conduite en traitant lesdites protéines phagiques avec une enzyme protéolytique.

5. Procédé selon la revendication 1, 2, 3 ou 4, caractérisé en ce qu'il comprend en outre les étapes d'ultrafiltration dudit filtrat obtenu dans la première étape à partir du filtre à membrane avant ladite étape de décomposition et de dénaturation des protéines phagiques.
